# EUROPEAN PATENT APPLICATION

(11) **EP 3 616 748 A1**
(43) Date of publication of application: **04.03.2020**
(21) Application number: 18190936.7
(22) Date of filing: 27.08.2018
(51) Int. Cl.: A61N 1/378, A61N 1/05, A61N 1/36, A61N 1/372, A61N 1/375, A61B 5/0488, A61B 5/04

(54) **IMPLANTABLE NEUROSTIMULATOR**

(71) Applicant: National University of Ireland, Galway, Galway (IE)
(72) Inventor: WARD, Fergal, Dublin, 4 (IE); LIDDY, Alison, Galway (IE); O'HALLORAN, Martin, Dublin, 4 (IE)
(74) Representative: FRKelly

(57) **Abstract**

Provided is an implantable neurostimulator comprising: a housing configured to be implanted into a patient; a microchip disposed in the housing, the microchip comprising a power antenna configured to receive power from an external source, a microcontroller configured to use the power to create and deliver neurostimulation signals, and a communication antenna configured to communicate with an external device; and at least one electrode configured to receive the neurostimulation signals and to apply the neurostimulation signals to target tissue.

## Description

### FIELD OF THE INVENTION

The present invention relates to an implantable neurostimulation device and a delivery system for delivering the implantable neurostimulation device into human tissue.

### BACKGROUND

Chronic post-operative pain is a condition that occurs following a surgical procedure due to peripheral nerve damage, with 450,000 people diagnosed with chronic postoperative pain every year in the U.S. Current treatments for peripheral nerve pain typically fall into two categories: drug or injectable. However neither of these categories is ideal for long term use. It is widely reported that drugs used to treat neuropathic pain can cause any of the following; kidney damage; liver damage; damage to the brain synapses plus these drugs are highly addictive. The injectable treatments are a massive cost burden on the health care provider where the patient needs to present every at least every three months for a treatment.

Neurostimulation is a proven therapy for peripheral neuropathic pain. New developments in the treatment of chronic neuropathic pain have led to the development of miniature neurostimulation implants such as described in EP1426 079, US7,711,419, US 8,810,405, US9,943,360 and US 2010/0060431. These implants can be placed at the target nerve and used as an alternative therapy for pain. However, none of these new implants are designed to meet the clinical need of the post-operative patient and all require cannulation to place the implant. It is not clinically recommended to cannulate in a postoperative patient as the area is tender and sore and has already undergone nerve and tissue damage and it is well reported that cannulation can cause tissue and nerve damage. Furthermore, the high cost of current commercially available neurostimulation implants-typically about $15,000-makes it an unavailable therapy for a lot pain patients. Consequently, neurostimulation devices are often only offered to patients after prolonged suffering where other treatment options have failed. Delivery systems for delivering implantable neurostimulators are described in US2004/0215133 and US 8,626,302.

### SUMMARY OF THE DISCLOSURE

The present disclosure provides an implantable neurostimulator that significantly reduces production costs and therefore makes it a product that is cost effective and can be considered as a first line treatment for pain. Also provided is a delivery system for delivering the implantable neurostimulator.

### BRIEF DESCRIPTION OF THE DRAWINGS

The novel features of the invention are set forth with particularity in the claims that follow. A better understanding of the features and advantages of the present disclosure will be obtained by reference to the following detailed description that sets forth illustrative embodiments, in which the principles of the present disclosure are utilized, and the accompanying drawings of which:
Figure 1 shows an implantable neurostimulator according to an embodiment of the present disclosure;
Figure 2A shows an implantable neurostimulator according to another embodiment of the present disclosure;
Figure 2B shows an external power source that can be used with the implantable neurostimulator of either Figure 1 or Figure 2A;
Figure 2C illustrates an implantable neurostimulator according to another embodiment of the present disclosure;
Figure 3 to 5 illustrate implantable neurostimulators according to embodiments of the present disclosure;
Figure 6 illustrates an external power source and external controller;
Figures 7 and 8 illustrate architectures of a microchip of an implantable neurostimulator according to embodiments of the present disclosure;
Figure 9 illustrates details of a sense channel component of the microchip of either Figure 7 or Figure 8;
Figures 10 and 11 show implantable neurostimulators employing two microchips incorporating power antennas in different planes, according to embodiments of the present disclosure;
Figures 12A and 12B illustrate an implant and delivery system according to an embodiment of the present disclosure;
Figure 13 shows an implantable neurostimulator similar to the embodiment shown in Figure 2A, modified to have a longer and more flexible connection between the electrode assembly and the housing, according to an embodiment of the present disclosure;
Figures 14-17 illustrate implant and single needle delivery systems according to embodiments of the present disclosure;
Figure 18 illustrates a delivery system comprising one needle in the form of a semi-circular shape with a second semi-circular shaped needle fitting into the lumen of the first needle to form a full circular shape, according to an embodiment of the present disclosure;
Figures 19-29 show a dual needle delivery system that may be used to deliver an implantable neurostimulator of the present disclosure, according to embodiments of the present disclosure;
Figures 30 and 31 illustrate implantable neurostimulators according to other embodiments of the present disclosure; and
Figure 32 illustrates a steerable needle with one or wires attached, according to another embodiment of the present disclosure.

### DETAILED DESCRIPTION

One aspect of the present disclosure is placement and incorporation of all design components required to produce a functioning implantable neurostimulator onto a microchip. Microchips once designed can be produced at a very low cost. By placing all of the active electronic design parts onto a single microchip the implant can be significantly reduced in size compared to traditional neurostimulation implants. This allows for the device to be placed minimally invasively, thereby reducing trauma to the surrounding tissue. The single chip may also integrate various passive electronic design parts, including but not limited to resistors, antenna and capacitors, in order to further reduce component counts and device cost.

Miniature neurostimulation implants can be placed in numerous different anatomical areas to treat peripheral nerve pain. The implantable neurostimulators of the present disclosure may be used, for example, to treat post operative nerve pain where the area has already been subjected to damage and requires an extremely minimally invasive procedure.

The present disclosure provides an implantable neurostimulator comprising:
a housing configured to be implanted into a patient; a microchip disposed in the housing, the microchip comprising a power antenna configured to receive power from an external source, a microcontroller configured to use the power to create and deliver neurostimulation signals, and a communication antenna configured to communicate with an external device; and at least one electrode configured to receive the neurostimulation signals and to apply the neurostimulation signals to target tissue.

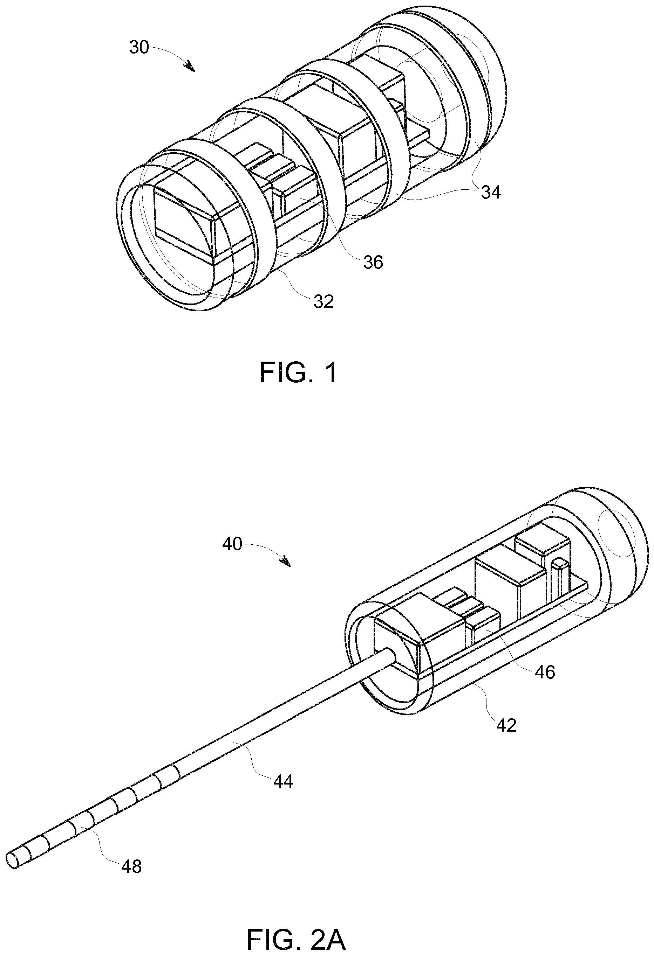

Figure 1 illustrates an implantable neurostimulator 30 according to an embodiment of the present disclosure. Referring to Figure 1, the implantable neurostimulator 30 has a housing 32 supporting electrodes 34. An outside surface of the housing 32 may be configured to be biocompatible with the tissue in which it will be implanted, and, except for exposed electrode areas, the housing 32 may be sealed to prevent ingress of body fluids. In some embodiments, the housing 32 has a width of approximately 3 mm and a length of approximately 9 mm. The housing 32 may be configured to fit inside a needle in the range of 16 to 8 gauge, although smaller needle sizes may be possible and desirable depending on the application.

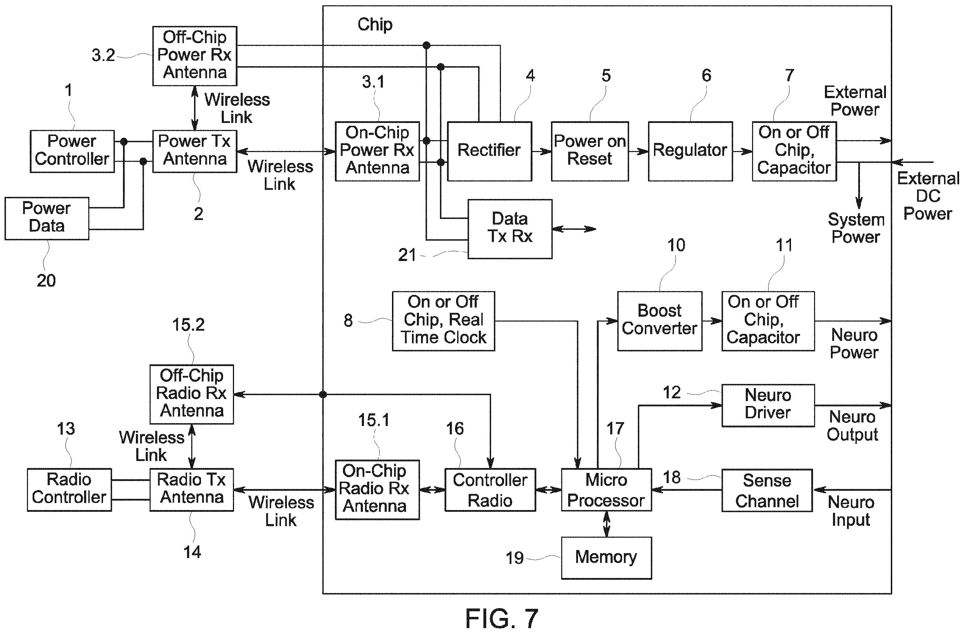

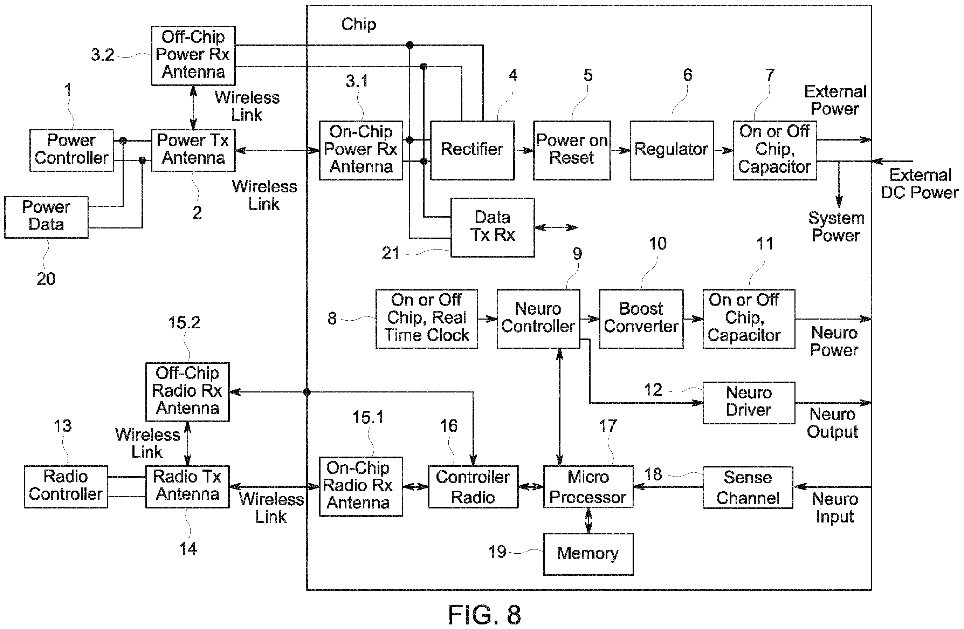

In the embodiment of Figure 1, disposed inside the housing 32 is a microchip 36 with neurostimulation electronics, a printed circuit board and discrete electronic components. One or more antennae may be incorporated into the microchip 36, into the printed circuit board and/or disposed as discrete components within the housing 32. The housing 32 may have more than one microchip. In addition, one or more antennae may be disposed within the housing 32 apart from the microchip 36. Details of the microchip architecture according to this embodiment are described below with reference to Figures 7 and 8.

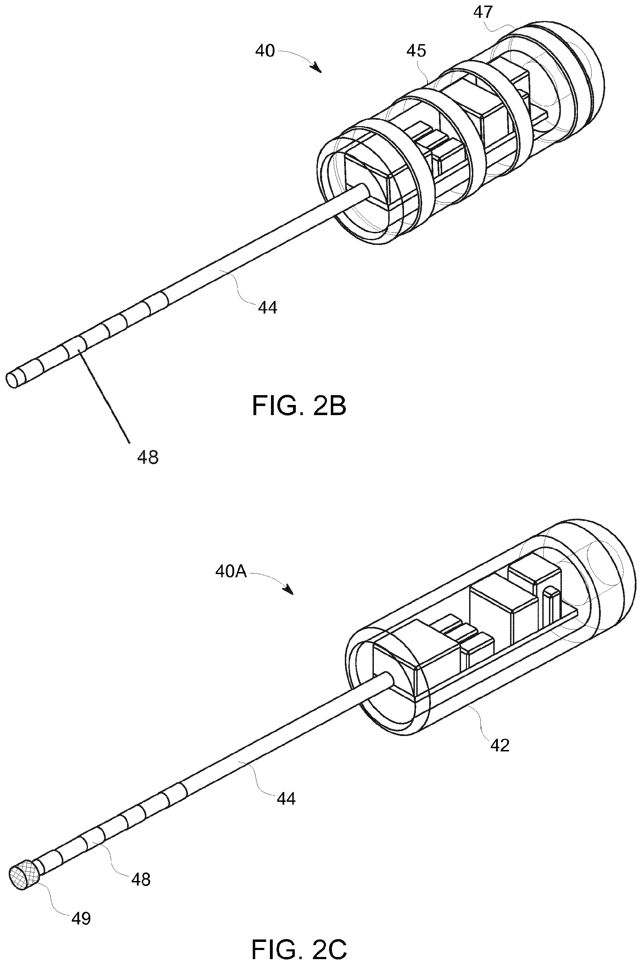

Figure 2A illustrates an implantable neurostimulator 40 according to another embodiment of the present disclosure. Referring to Figure 2A, the implantable neurostimulator has a housing 42 from which an electrode assembly 44 extends. The housing 42 comprises an opening to expose the electrode assembly 44 for applying the neurostimulation signals to the target tissue. The electrode assembly 44 comprises one or more electrodes 48. The electrode assembly 44 may be in the form of an elongated linear member extending from the housing 42. The one or more electrodes 48 may be located at different positions on the electrode assembly 44. The electrode assembly 44 has a much smaller diameter than the housing 42, enabling it to be placed at the desired stimulation site using a smaller access opening in the tissue than the opening required to place the housing 42. In this regard, the electrode assembly 44 may be configured to be delivered via a needle in the range of 23 to 17 gauge. Disposed inside the housing 42 is a microchip 46 with neurostimulation electronics, a printed circuit board and discrete electronic components. One or more antennae may be incorporated into the microchip 46, the printed circuit board and/or disposed as discrete components within the housing 42. In some embodiments, one or more antennae 45 may be incorporated into an outer packaging 47 surrounding the microchip 46, as shown in Figure 2B. The antennae 45 may be formed as a coil and may be embedded in the outer packaging material to form part of the cylindrical shape of the outer packaging. The microchip 46 may be configured to sense an Electromyography (EMG) signal and/or a Nerve Action Potential with the electrode assembly 44.

Figure 2C illustrates an implantable neurostimulator 40A according to another embodiment of the present disclosure. Referring to Figure 2C, a self-expanding anchor 49 at a distal end of an electrode assembly 44 may be delivered to the stimulation site in a compressed configuration (e.g., inside a delivery needle). The anchor 49 will then expand after deployment from the delivery needle to provide an anti-migration anchor for the implant, e.g., by providing a tissue-ingrowth site. The anchor 49 may be formed as a self-expanding braided mesh or self-expanding laser cut tube. The anchor 49 may be formed from a shape memory material such as nitinol and may be connected to the electrode assembly 44 by a wire.

In other embodiments (not shown), an anchor at the distal end of the electrode assembly 44 may be pre-coiled and pre-formed to form a hook after emerging from the delivery needle and expanding from its compressed delivery configuration to a deployed configuration. The anchor may be coated with a coating that promotes tissue overgrowth or etched to promote tissue overgrowth.

The electrode assembly 44 may have one or more exposed electrodes and is electrically connected to the printed circuit board via a conductor (not shown). In some other embodiments, the electrode assembly 44 may be separated from the housing 42 by a long flexible conductor to enable the electrode assembly 44 to be placed a distance away from the larger housing 42. The electrode assembly 44 and the long flexible conductor may be coiled in order to serve as an anti-migration feature.

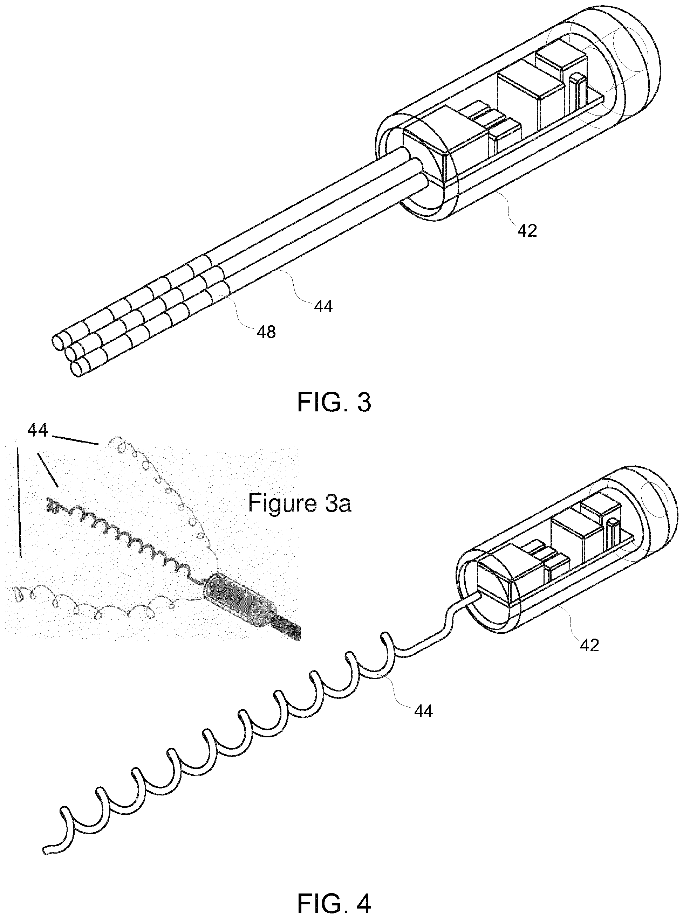

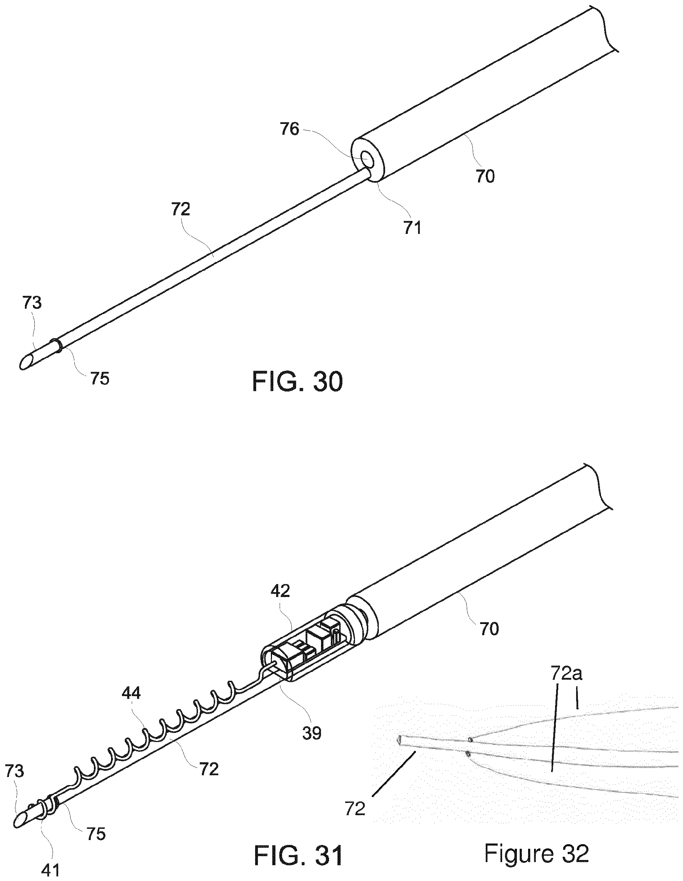

Figure 3 shows an embodiment in which a plurality of electrode assemblies 44, each with one or more electrodes 48, extend from the implant housing 42. The extent of the combined diameters of the electrode assemblies 44 may be configured to be less than the diameter of the housing 42, enabling the electrodes 48 to be placed at the desired stimulation site using a smaller access opening in the tissue than the opening required to place the housing 42. The plurality of electrode assemblies 44 may be configured to extend in different directions, as illustrated in Figure 3A, and as described later with reference to Figure 32. It will be understood that the plurality of electrode assemblies 44 extending in different directions may be one or more helical elements, as shown in Figure 3A, or linear electrode assemblies as per the embodiments of Figures 2A, 2B, 2C and 3.

Figure 4 shows an embodiment in which the electrode assembly 44 has one or more helical elements (with, e.g., constant or variable pitch geometry), each with one or more stimulating electrodes, extending from the housing 42. The helical shape of the electrode assembly 44 in this embodiment helps prevent migration of the electrodes after implantation.

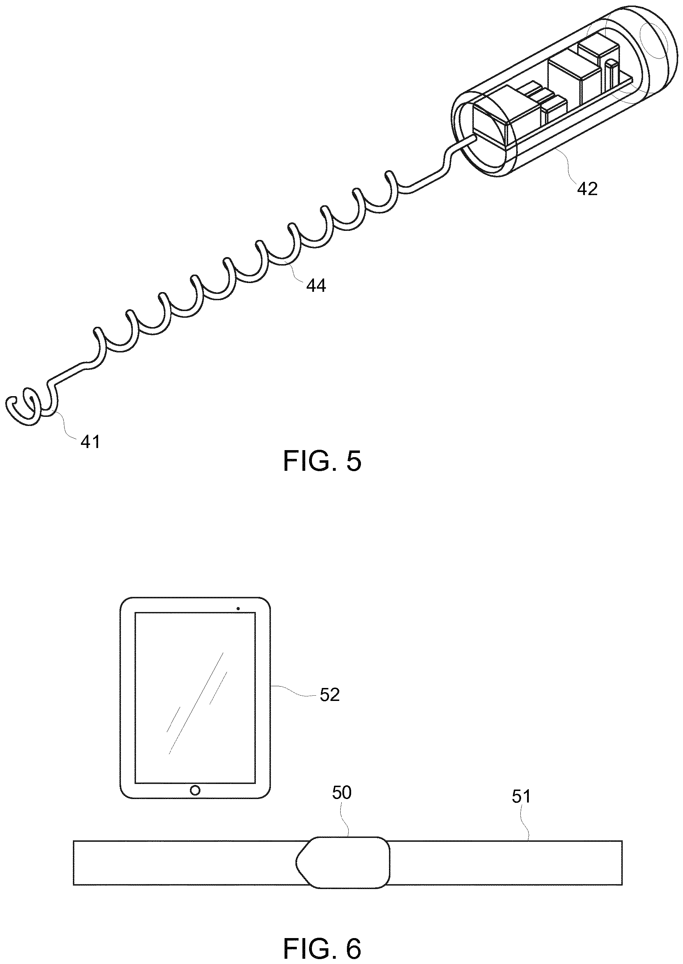

Figure 5 shows an embodiment in which the electrode assembly 44 has one or more helical elements (with, e.g., constant or variable pitch geometry), each with one or more stimulating electrodes, extending from the housing 42. The helical shape of the electrode assembly 44 in this embodiment helps prevent migration of the electrodes after implantation. An extended and offset helical portion 41 of the electrode assembly 44 grips or otherwise engages an implantation tool (such as a needle, not shown) during placement of the electrodes at the desired tissue stimulation site.

Figure 6 shows an external power source 50 and an external controller 52. The external power source 50 has one or more antennae (not shown) for transferring power to, and optionally communicating with, an implanted neurostimulator (such as those described above) when worn in a position external to the implant location. Referring to Figure 6, the external power source 50 may be supported by a strap 51 surrounding a portion of the patient's body.

The external controller 52 may run software enabling communication with one or more implanted neurostimulators of the present disclosure. The external controller 52 may be used to configure neurostimulation parameters and to transmit these parameters to the implantable neurostimulators and to the external power source 50. The external controller 52 may also be used to synchronize the stimulation delivered by multiple implanted neurostimulators to, e.g., enable symmetrical stimulation of nerves in left and right anatomical locations in order to provide symmetrical stimuli into the brain. The external controller 52 may also be used to separately configure one or more implanted neurostimulators into master/slave configurations for, e.g., power transfer capabilities. Implanted neurostimulators will only operate when powered by the external power source 50 and where one or both of the implantable neurostimulators and the external power source 50 are paired with the external controller 52 via Bluetooth (RTM) and/or other communications protocols.

Possible neurostimulation parameters include the following: up to 10.5V amplitude; up to 25 mA amplitude, pulse width 10 to 1000 µs, stimulation frequency 0.1 Hz to 50 kHz. The waveforms may be charge balanced. The waveform shape may be rectangular, rectangular in bursts, square, triangular, sinusoidal, Gaussian, sine-gaussian, stochastic or a PSD waveform. The stimulations may be pulsed, continuous, symmetrical, asymmetrical, monophasic waveforms, pseudo-monophasic waveforms, biphasic waveforms, or charge balanced waveforms. The stimulations may be in waveforms in a frequency range between 0.1 Hz and 100 kHz. The stimulations may be in waveforms having a current amplitude range between 0.1 mA and 25.0 mA. The stimulations may be in waveforms having a voltage amplitude range between 0.1 V and 12V. The stimulations may be in waveforms having a pulse width between 10 µs and 1000µs. The stimulations may be in waveforms having a pulse duty cycle.

In some embodiments, the external controller 52 may optionally communicate with an information and communication technology (ICT) system, including one or more computers running one or more software applications and having one or more databases and one or more networking devices and one or more storage systems and utilising one or more operations, administration and management tools for managing and maintaining the system. The ICT system may be a public or private or hybrid cloud-based system. The ICT system may be used to store device settings, to store stimulation settings of historical therapies, to provide recommendations for future therapy stimulation settings, to provide feedback to the patient and/or to the doctor, to provide notifications to the patient, to provide logging of daily pain episodes, and/or to provide logging of nerve bioelectrical activity. The ICT system may operate in conjunction with the external controller 52 via the network to provide the services. The system may use advanced analytics technologies and integrate all or some of the above data and/or indication data to recommend new therapy programs to the external controller 52 via the network. The ICT system may also monitor implanted neurostimulators to determine compliance with therapy prescriptions. The ICT system may also have the capability to deactivate an implanted electronic device whose implant indwell duration has expired.

Figure 7 shows schematically the architecture of the microchip of any of the implantable neurostimulator embodiments described above. A power controller 1 corresponds to the external power source 50 shown in Figure 6. The power controller 1 has one or more antennae to wirelessly transmit power to, and/or communicate information with, one or more antennae on the microchip 36 of the implantable neurostimulator or with an off-chip antenna within the implantable neurostimulator. A power antenna 2 may be either an inductor integrated into a silicon-based microchip 36 or, alternatively, as a discrete device via a wire wound coil or integrated into the printed circuit board. A rectifier 4 receives AC power from a power antenna 3.1 or 3.2 and converts it to DC power. A Power-On Reset block 5 is a chip component that generates a reset signal when power is applied to the device to ensure that the device starts operating in a known state. A regulator 6 is a chip component that communicates with the Power-On Reset block 5 and maintains a constant output voltage. A capacitor 6 smooths the delivery of system power.

Referring to Figure 8, a neurocontroller 9 is an optional component. When implemented, the neurocontroller 9 reduces the power usage of the system by turning off a microprocessor 17 during normal operation. Instead of using the power-hungry microprocessor 17 to control stimulation parameters, the microprocessor 17 programs the neurocontroller 9 to generate the output stimulus, and the microprocessor 17 then goes to sleep. The stimulation signal passes through a boost converter 10 and a capacitor 11, which limits the flow of DC current into the patient's body. Current and/or voltage regulated square and pulsed waveform outputs are delivered to the electrode(s) via the Neuro Power output. A neurodriver 12 sends the neurostimulation signal to the electrode(s) to stimulate the target tissue with current and/or voltage regulated sinusoidal waveforms via the Neuro Output.

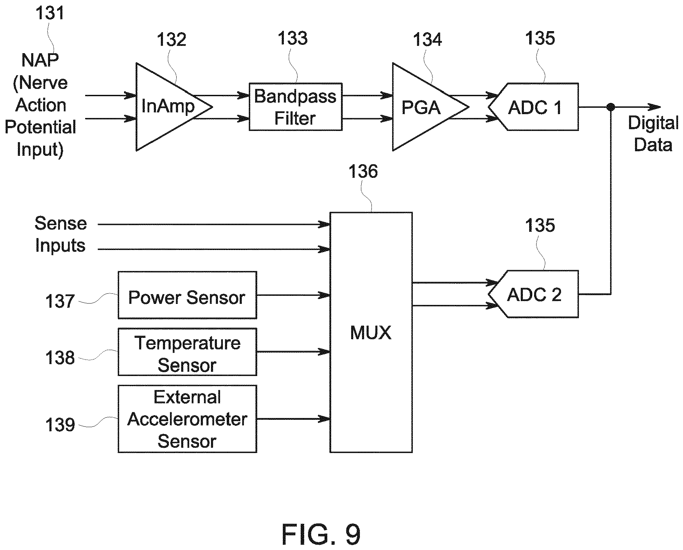

A radio antenna 15.1 on the microchip 36 or off-chip radio antenna 15.2 communicates with an external controller12 (such as the external controller 52 of Figure 6) and with a radio controller 13. A radio controller 16 communicates with the microprocessor 17. A sense channel 18 communicates with the microprocessor 17 to provide amplified sensor data from sensors associated with the implant, such as nerve bioelectric sensors, tissue temperature sensors, tissue bioimpedance sensors, stimulator output sensors and the like. Further details of the sense channel 18 are shown in Figure 9. Referring to Figure 9, a nerve action potential 131 is sensed, is amplified by an instrumentation amplifier 132, is filtered by a bandpass filter 133, is amplified by a programmable gain amplifier 134 and is digitised by a first analog to digital converter 135. Other sense inputs including, but not limited to a power sensor 137, a temperature sensor 138 and an external accelerometer sensor 139 are all combined together into a multiplexer 136 and then digitised by a second analog to digital converter 135 before and then transmitted to the microprocessor 17.

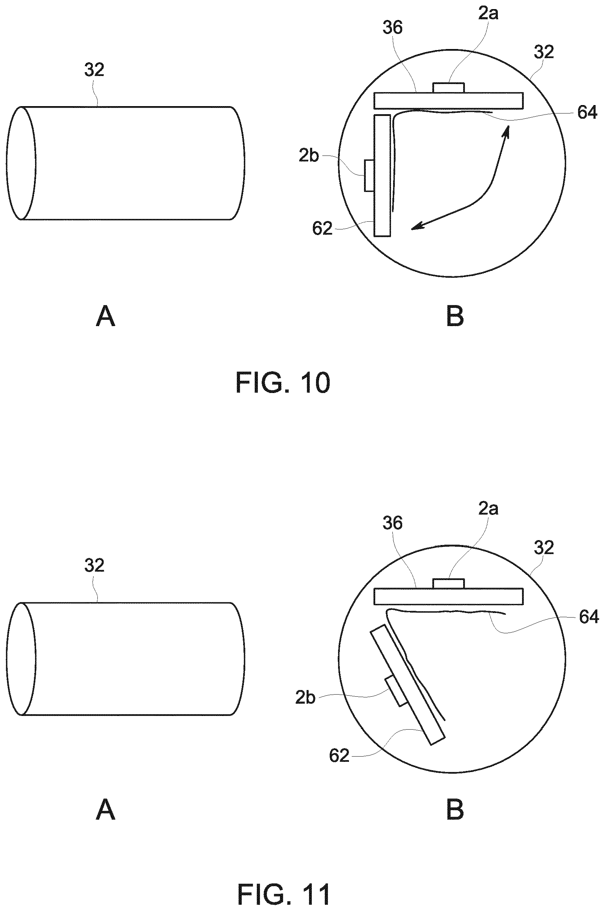

Figures 10 and 11 show implantable neurostimulators employing two microchips incorporating power antennas in different planes, according to embodiments of the present disclosure. Referring to Figures 10 and 11, a second microchip 62 is disposed in a housing 32 along with a first microchip 36 as described above. The second microchip 62 has a second power antenna 2b formed therein. The microprocessor 17 may control the second power antenna 2b on the second microchip 62 along with a first power antenna 2a on the first microchip 36 in a master/slave fashion. A conductor, such as a flexible circuit 64, connects the second microchip 62 to the first microchip 36. Having power receiving antennas in different planes reduces the need to orient the antenna of the external power source to be in the same plane as the implanted antenna. With two implanted power receiving antennas in two different planes, power may be harvested from the external power source in multiple planes. While Figure 10 shows the two microchips at approximately a 90° angle, other orientations are possible, as shown in Figure 11. In some embodiments, a third microchip (not shown) with a third integrated antenna may be provided inside the housing 32 and electrically connected to the first microchip 36 to be controlled in a master/slave arrangement.

The present disclosure also provides a delivery system configured for the placement of a miniature implantable neurostimulator without the need for cannulation, making it easy to place an implantable neurostimulator in a post-operative pain patient without any further damage occurring in the already damaged area. The delivery system of the present disclosure may be used to deliver an implantable neurostimulator of the present disclosure.

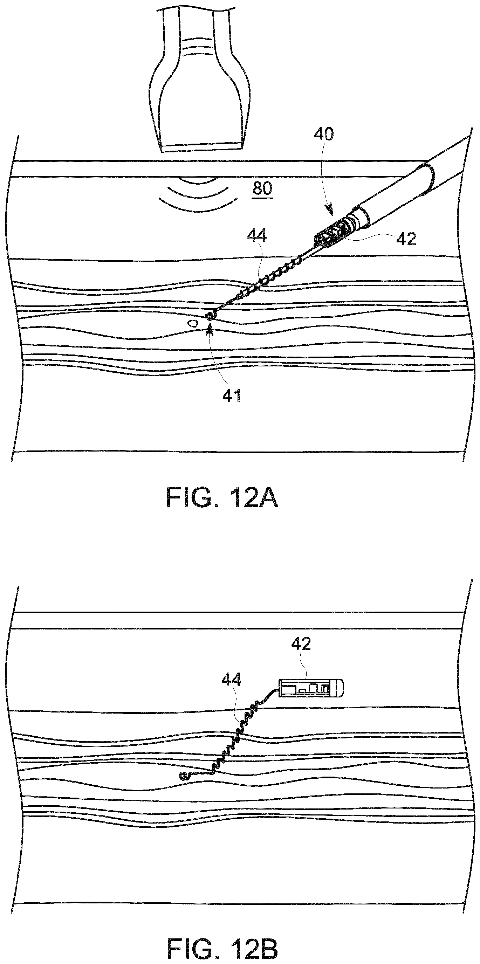

Figures 12A and 12B illustrate an implant and delivery system according to an embodiment of the present disclosure. Referring to Figures 12A and 12B, an implantable neurostimulator 40, similar to the embodiment shown in Figure 5, is configured to be placed at a stimulation site deep inside the patient's tissue. The housing 42 of the implantable neurostimulator 40 is disposed just below a patient's skin 80. This design minimizes the size of the implantation track at the stimulation site and the trauma to tissue between the skin surface and the implantation site during placement of the electrode assembly 44 and housing 42.

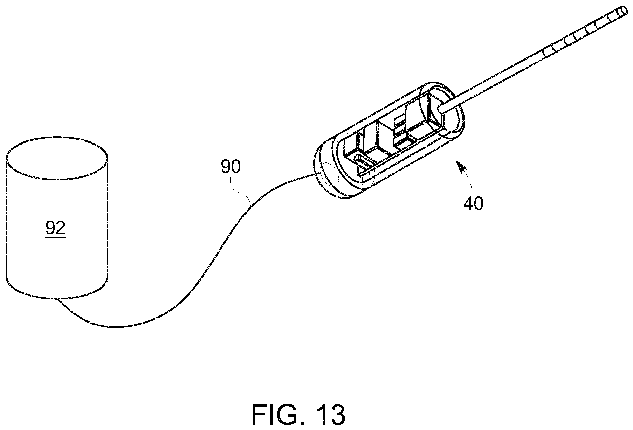

Figure 13 illustrates another embodiment of the present application in which the implantable neurostimulator (such as the implantable neurostimulator 40 described in Figure 2A) obtains power via a flexible conductor 90 extending through the patient's skin to an external power supply 92. Alternatively, the power may be obtained from a subcutaneous implanted power supply.

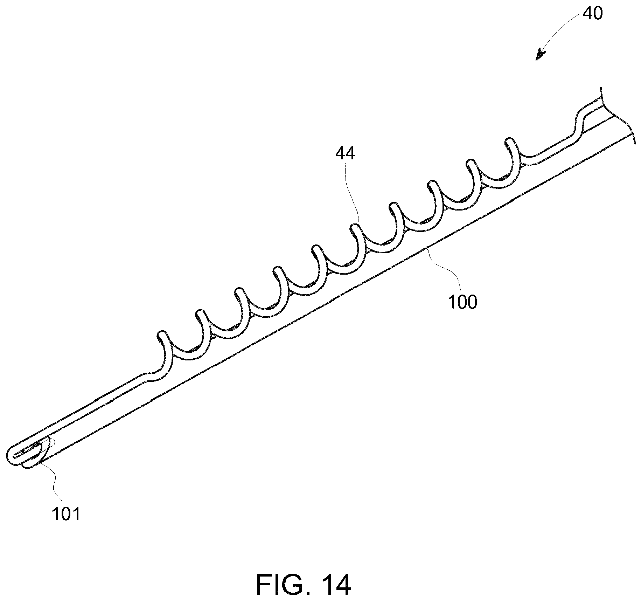

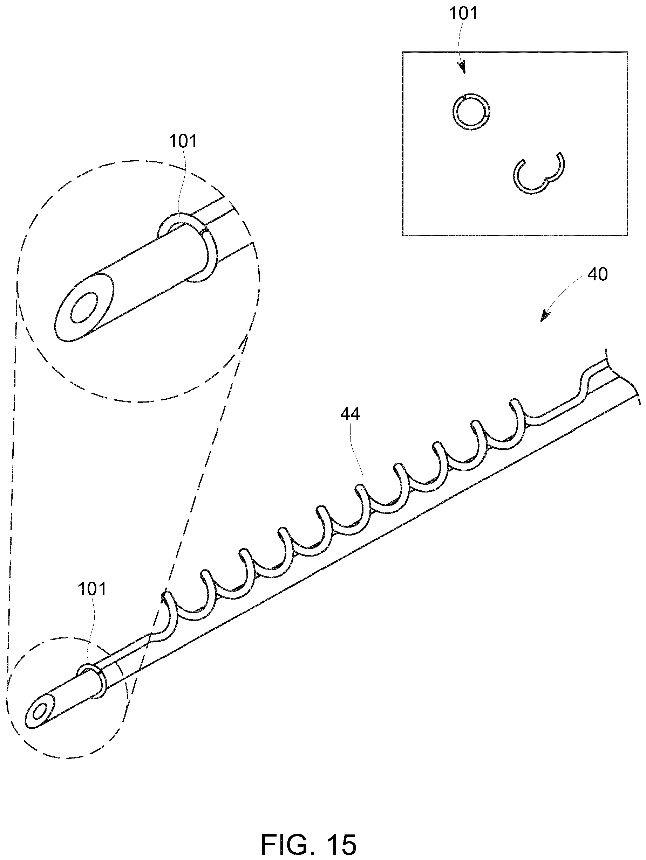

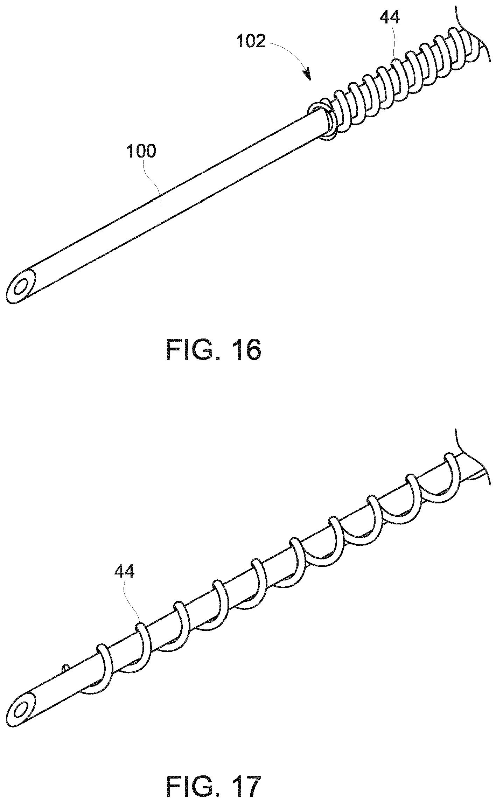

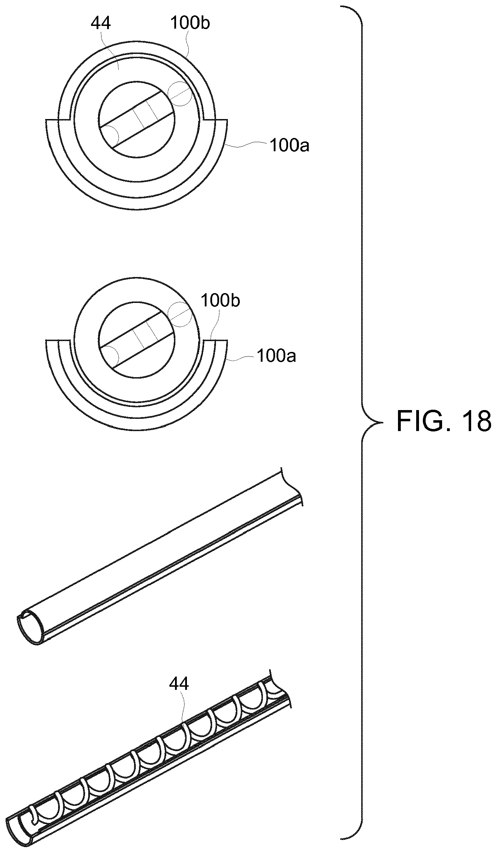

Figures 14-17 illustrate implant and single needle delivery systems according to embodiments of the present disclosure. The single needle delivery systems may be used to implant any of the implantable neurostimulators of the present disclosure, as described herein. Referring to Figures 14-17, the single needle delivery system comprises a single needle having a bore configured to pierce skin and extending from a needle support to implant an electrode of the implantable neurostimulator at a first tissue location. The single needle is configured to deliver the housing of the implantable neurostimulator to which the electrode is electrically connected to a second tissue location proximal to the first tissue location. In one embodiment, the electrode may be attached to the distal end of a needle by coiling it around the needle, as illustrated in Figures 17, 28, 29, and 31. In another embodiment, as illustrated in Figure 14, the electrode 44 of the implantable neurostimulator 40 can be fitted into an opening at the distal end of the needle 100 and pushed out from this space during the deployment. In another embodiment the electrode 44 can be fully loaded into a lumen of the needle 100 and pushed out during placement, as described below with reference to Figure 18. Referring to Figure 15, in another embodiment the electrode 44 of the implantable neurostimulator 40 may be attached using a clip 101 and the clip 101 opened to release the electrode 44 at the target site. Referring to Figures 16 and 17, in another embodiment the electrode 44 of the implantable neurostimulator 40 may be fully coiled around the needle 100. The electrode 44 may be tightly coiled around the needle 100 and held by a hook 102. The electrode 44 may be released by withdrawing the needle 100 and leaving the electrode 44 in place. Referring to Figure 16, the electrode 44 of the implantable neurostimulator 100 can be preloaded and compressed like a spring either outside or inside the lumen of the needle 100. Referring to Figure 17, the electrode 44 can then be released at the target site by removing the hook 102.

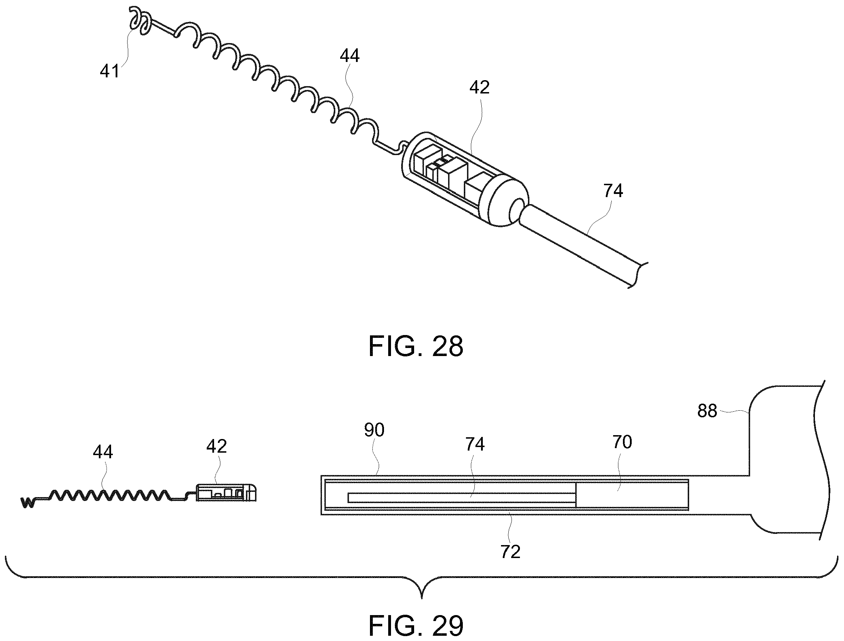

In the embodiments of Figures 14 to 17, the delivery system design consists of one small needle 100. In operation, a pain specialist creates a small incision beneath the skin and the larger part of the implantable neurostimulator 40 is positioned just beneath the skin. Next the small needle 100 is transitioned to the target site to deliver the electrode 44. The implantable neurostimulator 40 may be preloaded into an outer sheath and positioned beneath the skin using a pusher to push the implantable neurostimulator 40 from the outer sheath after an incision has been made. Following placement of the implantable neurostimulator 40 the needle 100 is transitioned to the target site. The outer sheath design may be bi-lumen and the delivery of the implantable neurostimulator 40 may be similar to that described with reference to Figure 28 below. The distal tip of the needle 100 may be bevel-shaped or any shape that enables penetration of skin and tissue.

Figure 18 illustrates a delivery system comprising a first needle 100a in the form of a semi-circular shape with a second semi-circular shaped needle 100b fitting into the lumen of the first needle 100a to form a full circular shape, according to an embodiment of the present disclosure. The first needle 100a may have a semi-circular shape or any curved shape that enables overlapping of two parts. The second needle 100b is then configured to fit into the lumen of the first needle 100a to form a full circular shape. The electrode 44 is loaded into the lumen of the first and second needles 100a, 100b combined. Once at the target site, the smaller second needle 100b is rotated inside the lumen of the larger first needle 100a. The electrode 44 is fully exposed and the needles are withdrawn. This design enables the electrode 44 to be fully encased as the needles are pushed through tissue and then exposed at the target site.

In another embodiment, the delivery system comprises a dual needle delivery system. The dual needle delivery system comprises a larger needle and a smaller needle. The larger needle may be a needle in the range of 16 to 8 gauge. The dual needle delivery system may have a needle support, the smaller diameter needle and the larger diameter needle. The larger needle is used for the housing containing the electronics and just penetrates tissue just beneath the skin. The larger needle is pushed just beneath the fatty tissue of the skin and the smaller needle is transitioned to the target site. The larger needle is used to deliver the larger part of the implantable neurostimulator whereas the smaller needle is used to deliver a small electrode to the target site. This design ensures that no cannulation is required. The smaller needle that is pushed deep into the tissue is as small as possible ensuring no tissue damage occurs. The needles can be made of any metal that is suitable for needle design. In one embodiment, the larger and smaller needles may be preloaded into an outer sheath.

In one embodiment, each of the smaller and larger needles may comprise one or more of the following: a polymer catheter with a needle tip attached; a laser cut hypotube with a needle tip attached; a braided or coiled sheath with a needle tip attached; or a metal needle design with a formed needle tip. The smaller and larger needles may be controlled by a handle to twist and rotate in the X, Y, or Z axis. This can be achieved by attaching wires to the sides of the needles and operating these wires from a handle of the device. In some embodiments the surface of both needles may be configured to reduce surface friction. This may be achieved by applying a coating, etching and/or reducing the surface area for example by creating bumps on the surface.

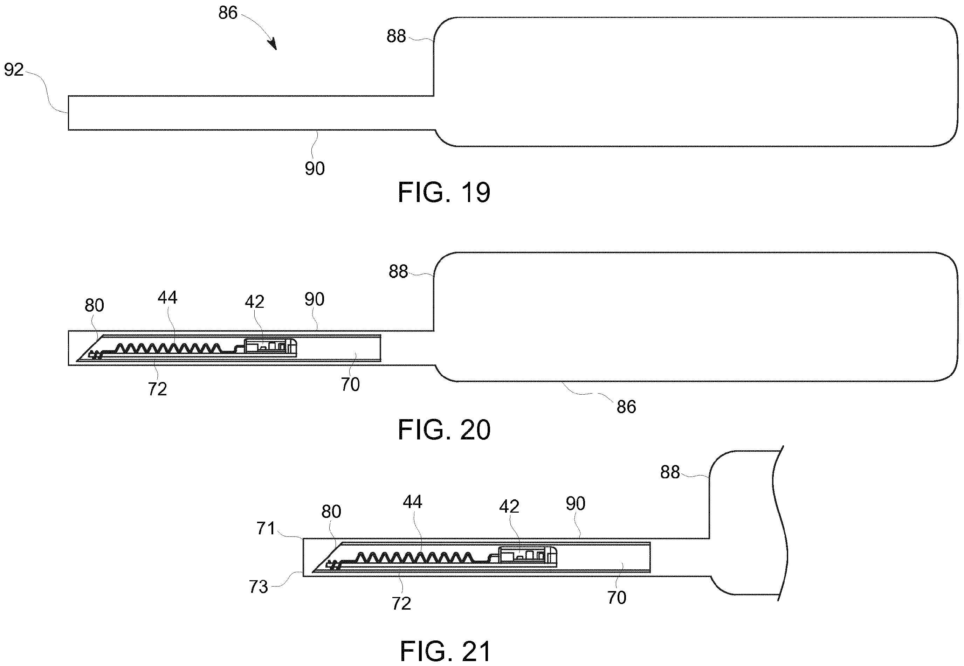

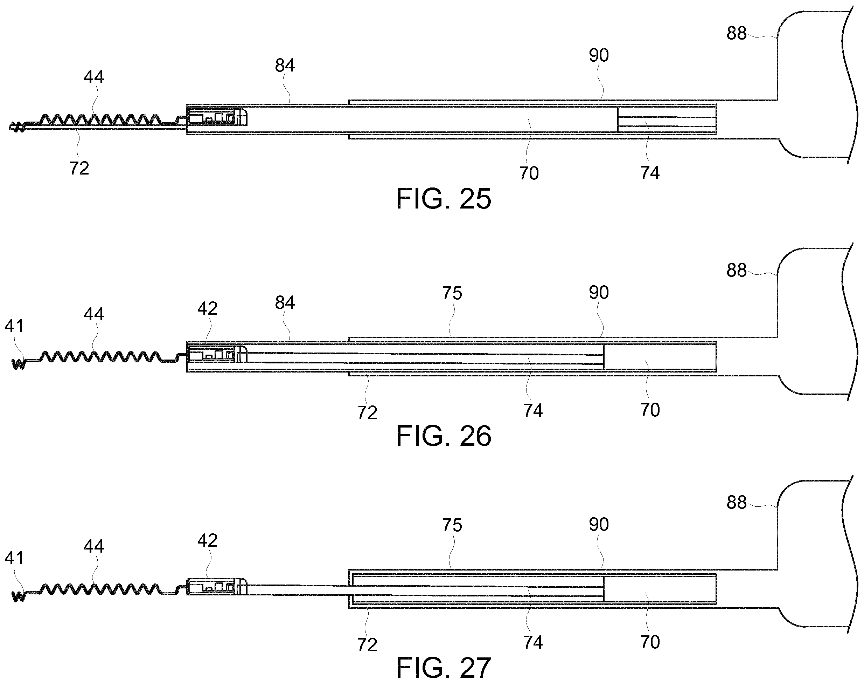

Figures 19-29 show a dual needle delivery system that may be used to deliver an implantable neurostimulator of the present disclosure, according to embodiments of the present disclosure. The dual needle delivery system comprises a second larger needle having a larger diameter than a first smaller needle, the second larger needle configured to contain the housing and to penetrate tissue just beneath the skin, the first smaller needle being configured to deliver the at least one electrode to the first tissue location. Referring to Figure 19, the dual needle delivery system includes an actuation system 86 having a handle 88, a delivery sheath 90 and actuation controls (not shown). To deploy an implantable neurostimulator, a distal end 92 of the delivery sheath 90 is placed against the patient's skin at the implant entry site. Referring to Figure 20, the dual needle delivery system has a small diameter needle 72 extending from a needle support 70 in the delivery sheath 90. As shown in Figure 21, a proximal end of the housing 42 of the implantable neurostimulator abuts a distal end 71 of the needle support 70 prior to deployment. The small diameter needle 72 has a sharp distal tip 73. The small diameter needle 72 may thus be passed through skin and other intervening tissue to place the electrode assembly at the target site without the need to extend a larger diameter needle 80 or the needle support 70 all the way to the target site. In some embodiments, placement of the electrode assembly may be conducted under ultrasound guidance. The small diameter needle 72 may be in the range of 23 to 17 gauge. Referring to Figures 26 and 27, a plunger 74 may be disposed in a bore 75 of the needle support 70. A distal end of the plunger 74 may be removably attached to the housing 42 prior to implantation. After placement, the small diameter needle 72 may be withdrawn, e.g., by operating the handle 88 on the delivery system. After placement of the electrode assembly, the larger diameter needle 80 with a sharp distal end 82 may be inserted through the skin over the small diameter needle 72 to place the housing (such as housing 42 described above) containing the neurostimulator microchip and integrated electronics at a shallower location, e.g., just beneath the patient's skin, by operating the plunger 74. The needles are withdrawn from the patient after implantation of the implantable neurostimulator. The larger diameter needle 80 may be in the range of 16 to 8 gauge.

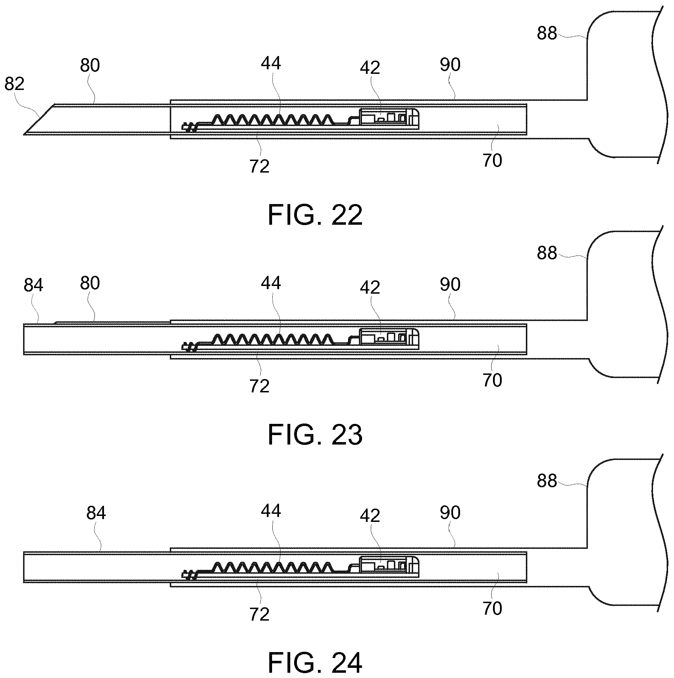

Referring to Figures 23 and 24, the dual needle delivery system also includes an introducer 84. In operation, the sharp distal end 82 of the larger diameter needle 80 is advanced out of the delivery sheath 90 into the patient's skin, as shown in Figure 22, using the handle 88. The introducer 84 is then advanced through the larger diameter needle 80 and over the housing 42 and electrode assembly 44 of the implantable neurostimulator. The larger diameter needle 80 is withdrawn, as shown in Figures 23 and 24. The smaller diameter needle 72 and implantable neurostimulator are then advanced through the introducer 84 by advancing the needle support 70 and the plunger 74, as shown in Figure 25. The smaller diameter needle 72 is then retracted from the implantable neurostimulator by retracting the needle support 70 without retracting the plunger 74, as shown in Figure 26. The introducer 84 is then retracted, and the plunger 74 is detached from the housing 42, as shown in Figures 27-29, to complete the deployment of the implantable neurostimulator. Use of a smaller diameter needle to advance the electrode assembly 44 and a larger diameter needle to inject the larger housing 42 enables placement of the electrodes at the stimulation site using the smallest possible implantation track.

In some embodiments, the implant placement procedure described above may be performed under ultrasound guidance. The implant and dual needle delivery system of Figures 19-29 may be used to deliver implantable neurostimulators to treat post-operative pain. In such patients, it may not be desirable to cannulate the tissue all the way to the target electrode implant site. This implant design and delivery system avoids the need for cannulation with a large bore needle to place the electrodes. The larger bore needle is used only for the larger housing containing the electronics. The dual needle delivery system as described with reference to Figures 19-29 may be used to implant any of the implantable neurostimulators of the present disclosure, as described herein.

In one embodiment, each of the smaller and larger needles of the dual needle delivery system described above may be configured to transition in the same direction from a lumen of an outer sheath, as illustrated in Figures 25-27. In one embodiment the smaller needle may be configured to transition from the lumen of the larger needle and operate in the same direction or a different direction to that of the larger needle. In another embodiment, the delivery system may comprise a multi-needle delivery system. In such a multi-needle delivery system the system may be configured to operate the same as the above described delivery system but multiple needles may be loaded in the handle. Each needle may be used to house a respective electrode assembly. A first needle may be removed to deliver the first electrode, and then a second needle may be tracked over the same transition path as the first needle. The multiple needles may be configured to operate in different directions to each other, for example to deliver the implantable neurostimulator of Figure 3A. In this regard, needles housing the electrodes may be transitioned to different locations via steerable mechanics/electro-mechanics. In this regard, Figure 32 illustrates a steerable needle 72 with one or wires 72a attached, according to another embodiment of the present disclosure. The one or wires 72a may be controlled from the handle of the delivery system to steer the needle 72 in different directions. In this manner, an electrode assembly contained within the lumen of the needle 72 may be delivered to a target area. The dual or multi-needle delivery system described above may be employed to deliver any of the implantable neurostimulators of the present disclosure as described herein.

Figures 30 and 31 illustrate an implantable neurostimulator and delivery system according to another embodiment of the present disclosure. In this embodiment, the implantable neurostimulator is similar to that shown in Figure 5, but with a housing 42 having a flat side 39, as shown in Figure 31. The delivery system has a small diameter needle 72 extending from a needle support 70. The flat side 39 of the housing 42 is disposed lengthwise on the small diameter needle 72. The proximal end of the housing 42 of the implantable neurostimulator abuts the distal end 71 of the needle support 70 prior to deployment. The small diameter needle 72 has a sharp distal tip 73. As stated above, the small diameter needle 72 may be in the range of 23 to 17 gauge. An extended and offset helical portion 41 of the electrode assembly 44 engages a distal portion of the small diameter needle 72 distal to a stop element 75. A plunger is disposed in a bore 76 of the needle support 70. A distal end of the plunger may be removably attached to the housing 42 prior to implantation.

When a feature or element is herein referred to as being "on" another feature or element, it can be directly on the other feature or element or intervening features and/or elements may also be present. In contrast, when a feature or element is referred to as being "directly on" another feature or element, there are no intervening features or elements present. It will also be understood that, when a feature or element is referred to as being "connected", "attached" or "coupled" to another feature or element, it can be directly connected, attached or coupled to the other feature or element or intervening features or elements may be present. In contrast, when a feature or element is referred to as being "directly connected", "directly attached" or "directly coupled" to another feature or element, there are no intervening features or elements present. Although described or shown with respect to one embodiment, the features and elements so described or shown can apply to other embodiments. It will also be appreciated by those of skill in the art that references to a structure or feature that is disposed "adjacent" another feature may have portions that overlap or underlie the adjacent feature.

Terminology used herein is for the purpose of describing particular embodiments only and is not intended to be limiting of the invention. For example, as used herein, the singular forms "a", "an" and "the" are intended to include the plural forms as well, unless the context clearly indicates otherwise. It will be further understood that the terms "comprises" and/or "comprising," when used in this specification, specify the presence of stated features, steps, operations, elements, and/or components, but do not preclude the presence or addition of one or more other features, steps, operations, elements, components, and/or groups thereof. As used herein, the term "and/or" includes any and all combinations of one or more of the associated listed items and may be abbreviated as "/".

Spatially relative terms, such as "under", "below", "lower", "over", "upper" and the like, may be used herein for ease of description to describe one element or feature's relationship to another element(s) or feature(s) as illustrated in the figures. It will be understood that the spatially relative terms are intended to encompass different orientations of the device in use or operation in addition to the orientation depicted in the figures. For example, if a device in the figures is inverted, elements described as "under" or "beneath" other elements or features would then be oriented "over" the other elements or features. Thus, the exemplary term "under" can encompass both an orientation of over and under. The device may be otherwise oriented (rotated 90 degrees or at other orientations) and the spatially relative descriptors used herein interpreted accordingly. Similarly, the terms "upwardly", "downwardly", "vertical", "horizontal" and the like are used herein for the purpose of explanation only unless specifically indicated otherwise.

Although the terms "first" and "second" may be used herein to describe various features/elements (including steps), these features/elements should not be limited by these terms, unless the context indicates otherwise. These terms may be used to distinguish one feature/element from another feature/element. Thus, a first feature/element discussed below could be termed a second feature/element, and similarly, a second feature/element discussed below could be termed a first feature/element without departing from the teachings of the present invention.

Throughout this specification and the claims which follow, unless the context requires otherwise, the word "comprise", and variations such as "comprises" and "comprising" means various components can be co-jointly employed in the methods and articles (e.g., compositions and apparatuses including device and methods). For example, the term "comprising" will be understood to imply the inclusion of any stated elements or steps but not the exclusion of any other elements or steps.

As used herein in the specification and claims, including as used in the examples and unless otherwise expressly specified, all numbers may be read as if prefaced by the word "about" or "approximately," even if the term does not expressly appear. The phrase "about" or "approximately" may be used when describing magnitude and/or position to indicate that the value and/or position described is within a reasonable expected range of values and/or positions. For example, a numeric value may have a value that is +/- 0.1% of the stated value (or range of values), +/- 1% of the stated value (or range of values), +/- 2% of the stated value (or range of values), +/- 5% of the stated value (or range of values), +/- 10% of the stated value (or range of values), etc. Any numerical values given herein should also be understood to include about or approximately that value, unless the context indicates otherwise. For example, if the value "10" is disclosed, then "about 10" is also disclosed. Any numerical range recited herein is intended to include all sub-ranges subsumed therein. It is also understood that when a value is disclosed that "less than or equal to" the value, "greater than or equal to the value" and possible ranges between values are also disclosed, as appropriately understood by the skilled artisan. For example, if the value "X" is disclosed the "less than or equal to X" as well as "greater than or equal to X" (e.g., where X is a numerical value) is also disclosed. It is also understood that the throughout the application, data is provided in a number of different formats, and that this data, represents endpoints and starting points, and ranges for any combination of the data points. For example, if a particular data point "10" and a particular data point "15" are disclosed, it is understood that greater than, greater than or equal to, less than, less than or equal to, and equal to 10 and 15 are considered disclosed as well as between 10 and 15. It is also understood that each unit between two particular units are also disclosed. For example, if 10 and 15 are disclosed, then 11, 12, 13, and 14 are also disclosed.

Although various illustrative embodiments are described above, any of a number of changes may be made to various embodiments without departing from the scope of the invention as described by the claims. For example, the order in which various described method steps are performed may often be changed in alternative embodiments, and in other alternative embodiments one or more method steps may be skipped altogether. Optional features of various device and system embodiments may be included in some embodiments and not in others. Therefore, the foregoing description is provided primarily for exemplary purposes and should not be interpreted to limit the scope of the invention as it is set forth in the claims.

The examples and illustrations included herein show, by way of illustration and not of limitation, specific embodiments in which the subject matter may be practiced. As mentioned, other embodiments may be utilized and derived there from, such that structural and logical substitutions and changes may be made without departing from the scope of this disclosure. Such embodiments of the inventive subject matter may be referred to herein individually or collectively by the term "invention" merely for convenience and without intending to voluntarily limit the scope of this application to any single invention or inventive concept, if more than one is, in fact, disclosed. Thus, although specific embodiments have been illustrated and described herein, any arrangement calculated to achieve the same purpose may be substituted for the specific embodiments shown. This disclosure is intended to cover any and all adaptations or variations of various embodiments. Combinations of the above embodiments, and other embodiments not specifically described herein, will be apparent to those of skill in the art upon reviewing the above description.

## Claims

1. An implantable neurostimulator comprising:
a housing configured to be implanted into a patient;
a microchip disposed in the housing, the microchip comprising a power antenna configured to receive power from an external source, a microcontroller configured to use the power to create and deliver neurostimulation signals, and a communication antenna configured to communicate with an external device; and
at least one electrode configured to receive the neurostimulation signals and to apply the neurostimulation signals to target tissue.

2. The implantable neurostimulator of claim 1, wherein the housing is sized and configured to be contained within a needle in the range of 16 to 8 gauge.

3. The implantable neurostimulator of claim 1 or 2, wherein the at least one electrode is disposed in the housing, the housing comprising an opening to expose the at least one electrode for applying the neurostimulation signals to the target tissue.

4. The implantable neurostimulator of claim 3, comprising a plurality of electrodes disposed in the housing, the housing comprising a plurality of openings to expose the electrodes for application of the neurostimulation signals to the target tissue.

5. The implantable neurostimulator of claim 1 or 2, wherein the at least one electrode is disposed outside of the housing and the neurostimulator further comprises a flexible electrical connection extending from the microchip to the at least one electrode.

6. The neurostimulator of claim 5 wherein the at least one electrode is configured to be delivered via a needle in the range of 23 to 17 gauge.

7. The neurostimulator of any of the preceding claims, wherein the microchip further comprises a neurocontroller configured to be configured by the microcontroller to deliver a neurostimulation signal, the microcontroller being configured to power down after configuring the neurocontroller.

8. The neurostimulator of any of the preceding claims wherein the microchip is further configured to sense an Electromyography (EMG) signal and/or a Nerve Action Potential with the at least one electrode.

9. The neurostimulator of any of the preceding claims, wherein the power antenna comprises a first power antenna, the neurostimulator further comprising a second power antenna disposed in the housing, configured to receive power from the external source and operatively connected to the microchip, the first power antenna and the second power antenna being disposed in different planes.

10. The neurostimulator of claim 9, wherein the microchip is operatively connected to the first and second power antennas and configured to use the power to create and deliver neurostimulation signals.

11. The neurostimulator of claim 9 or 10, wherein the communication antenna is disposed on the microchip.

12. The neurostimulator of claim 10 or 11, wherein the first power antenna is disposed on the microchip.

13. The neurostimulator of claim 12, wherein the microchip comprises a first microchip, the neurostimulator further comprising a second microchip disposed in the housing, the second power antenna being disposed on the second microchip.

14. A delivery system for delivering the implantable neurostimulator of any of preceding claim, the delivery system comprising a first needle having a bore configured to pierce skin and extending from a needle support to implant an electrode of the implantable neurostimulator at a first tissue location, the first needle being configured to deliver the housing of the implantable neurostimulator to which the at least one electrode is electrically connected to a second tissue location proximal to the first tissue location.

15. The delivery system of claim 14, comprising a second needle having a larger diameter than the first needle, the second needle configured to contain the housing and to penetrate tissue just beneath the skin, the first needle being configured to deliver the at least one electrode to the first tissue location.
